Europäisches Patentamt

European Patent Office

Office européen des brevets

(11) Numéro de publication: **0 396 536 B1**

# (12) FASCICULE DE BREVET EUROPEEN

(45) Date de publication de fascicule du brevet: **31.08.94**   (51) Int. Cl.⁵: **A61M 5/14**

(21) Numéro de dépôt: **88900828.0**

(22) Date de dépôt: **07.01.88**

(86) Numéro de dépôt internationale :
**PCT/FR88/00012**

(87) Numéro de publication internationale :
**WO 89/06145 (13.07.89 89/15)**

(54) **DISPOSITIF POUSSE-SERINGUE AMBULATOIRE POUR INJECTIONS PARENTERALES A DEBIT ASSERVI AU CONTENU DE LA SERINGUE ET SON PROCEDE DE CONTROLE.**

(43) Date de publication de la demande:
**14.11.90 Bulletin  90/46**

(45) Mention de la délivrance du brevet:
**31.08.94 Bulletin  94/35**

(84) Etats contractants désignés:
**AT BE CH DE FR GB IT LI LU NL SE**

(56) Documents cités:
**EP-A- 0 016 343**
**US-A- 4 662 872**

(73) Titulaire: **HAZON, Bernard**
**5 avenue de l'Opéra**
**F-75001 Paris (FR)**

(72) Inventeur: **HAZON, Bernard**
**5 avenue de l'Opéra**
**F-75001 Paris (FR)**
Inventeur: **SAUSSE, Andre**
**12, avenue Franklin Roosevelt**
**F-92330 Sceaux (FR)**

(74) Mandataire: **Hasenrader, Hubert et al**
**Cabinet Beau de Loménie**
**158, rue de l'Université**
**F-75340 Paris Cédex 07 (FR)**

EP 0 396 536 B1

## Description

La présente invention a pour objet un dispositif pousse-seringue pour injection intraveineuse ambulatoire en volumes élémentaires ou bolus séparés par des périodes fixes de relaxation, par asservissement du bolus au volume quelconque de fluide contenu dans une seringue indifférenciée, et un procédé de contrôle du débit moyen assuré par un tel dispositif.

L'avantage essentiel du dispositif précité consiste en ce qu'il ne nécessite aucune programmation par l'utilisateur, supprimant ainsi les risques inhérents à tout calcul et/ou dilution préalable(s).

Les dispositifs pousse-seringue selon l'art antérieur utilisent en général un moyen actif de translation du piston de la seringue dont le corps est fixé sur l'appareil.

Parmi les moyens de translation, on trouve le plus souvent un chariot et une vis-sans-fin actionnée par un moteur électrique.

Le moteur qui actionne la vis-sans-fin est soit du type pas-à-pas, soit à courant continu. Dans ce dernier cas un réducteur est en général placé entre le moteur et la vis-sans-fin voir par exemple le brevet US-A-4 662 872.

Pour une seringue géométriquement définie, le débit de solution injectée est directement proportionnel à la vitesse de translation de l'organe pousse-piston, ladite vitesse étant à son tour directement proportionnelle à la vitesse de rotation angulaire de la vis-sans-fin.Le réglage de la vitesse angulaire de la vis-sans-fin peut s'opérer en rotation continue, par exemple en contrôlant l'alimentation du moteur électrique.

Plus récement on a préféré une rotation discontinue de la vis-sans-fin, au moyen de périodes actives séparées par des périodes de relaxation. Il est ainsi distribué pendant les périodes actives des volumes élémentaires ou bolus. On obtient par ce moyen une plage de variation de la vitesse angulaire moyenne de la vis-sans-fin, donc du débit moyen, beaucoup plus étendue, par exemple de 1 à 100.

Comme dans le brevet US-A-4 662 872, un programmateur, en général une horloge, initie la rotation de la vis-sans-fin, rotation qui se termine après description d'un angle prédéterminé et constant.

Une variante récente consiste à remplacer la programmation de l'arrêt par un asservissement qui arrête le moteur lorsque l'angle nécessaire et suffisant à la délivrance d'un bolus a été décrit. Les variations de vitesse du moteur sont alors sans influence sur la précision volumétrique du bolus.

Le réglage du débit est obtenu par variation de la fréquence des initiations de rotation de la vis-sans-fin, ce qui s obtient en général en faisant varier la durée de la relaxation dans la mesure ou celle-ci reste grande devant la durée des périodes actives alors considérée comme négligeable. Ces modes de réglage de la vitesse de translation de l'organe pousse-piston présentent des inconvénients.

En effet, la posologie étant généralement exprimée en moles ou unités de produit actif par kilogramme de poids corporel et par jour, sa traduction en vitesse de translation du piston de la seringue nécessite des calculs préalables. En général, un seul type de seringue peut-être utilisé. Ou encore, il est nécessaire d'employer des tables de conversion permettant d'afficher le débit en fonction du type de seringue utilisée.

De plus, les durées de relaxation nécessaires avec des débits faibles obtenus avec des seringues de trop grande capacité relative peuvent être incompatibles avec la demi-vie de la drogue injectée et/ou avec le maintien de la perméabilité au moyen d'injection intra-vasculaire.

En outre, la durée de vidange totale de la seringue est variable, ce qui rend l'horaire de renouvellement de son contenu différent d'un patient à l'autre.

Enfin, la marche continue du moteur est en général trop lente pour permettre le retour arrière du chariot autrement que par débrayage dudit chariot et poussée manuelle.La présente invention a pour but de résoudre les problèmes précédents.

Ce but est atteint au moyen d'un dispositif pousse-seringue pour traitement ambulatoire au moyen d'une seringue indifférenciée munie d'un piston délivrant une pluralité de volumes élémentaires ou bolus séparés par des périodes de relaxation, comprenant :

- un boîtier dans lequel est située une enveloppe-guide fermée contenant un ensemble électromécanique composé d'un moteur à faible inertie sans fer tournant, à excitation par aimant permanent,
- un réducteur,
- une vis-sans-fin coaxiale sur laquelle se déplace un chariot porteur d'un organe pousse-piston,
- une source d'énergie électrique, et,
- des circuits électroniques de contrôle avec au moins un compteur,
  caractérisé en ce qu'il comprend en outre :
- un premier compteur-décompteur réversible qui intègre les tours positifs et négatifs du moteur,
- un second compteur préconfigurable,
- un moyen qui divise la longueur totale de la course en un segment avant et un segment arrière,
- un moyen de détection du contact entre l'organe pousse-piston et le piston,

- un circuit capable de mettre à zéro le premier compteur-décompteur lors de la détection d'un contact entre l'organe pousse-piston et le piston,
- un circuit capable de transmettre, lors de la détection d'un contact entre l'organe pousse-piston et le piston dans le segment arrière, la lecture du compteur-décompteur réversible au compteur préconfigurable après division préalable par le produit nP du nombre total de bolus n et d'un facteur P fonction à la fois du rapport de réduction du réducteur et du nombre de signaux émis par tour de moteur,
- un circuit qui permet de diriger les signaux provenant de la rotation du moteur sur les deux compteurs à la fois,
- une horloge qui permet d'initialiser la rotation du moteur au terme d'un temps égal à la période de cette horloge,
- un circuit qui permet d'arrêter la rotation du moteur lorsque la valeur comptée par le premier compteur et la valeur stockée dans la mémoire du compteur coïncident.

Un autre objet de l'invention est un procédé de contrôle du débit moyen assuré par un dispositif pousse-seringue motorisé du type précédent.

Ce procédé est caractérisé en ce qu'il comprend une phase de tarage préalable telle que :

a) la seringue est placée vide dans le dispositif

b) l'organe pousse-seringue est déplacé vers l'avant jusqu'à l'obtention du contact avec le piston

c) le premier compteur est remis à zéro lorsque ledit contact survient dans la portion courte avant de la course mécanique de l'organe pousse-piston, provoquant la coupure générale de l'alimentation du dispositif, à l'exception de la marche arrière rapide à commande manuelle

d) l'organe pousse-piston est renvoyé dans le segment long arrière pour permettre l'introduction de la seringue contenant la dose D de fluide à injecter

e) l'organe pousse-piston est renvoyé en marche avant et entre en contact avec le piston de la seringue dans la portion longue arrière du dispositif de façon à activer une bascule bistable qui provoque :

f) l'arrêt de l'alimentation du moteur via l'avance rapide

g) le transfert de la lecture du premier compteur, préalablement divisée par nP correspondant au produit du nombre total de bolus n par un facteur P fonction du rapport de réduction du réducteur et du nombre de signaux émis par tour ou fraction de tour du moteur, dans la mémoire d'un deuxième compteur, préconfigurable

h) la fermeture d'un circuit tel que les signaux à provenir de la rotation du moteur seront ultérieurement dirigés, parallèlement au premier compteur vers le deuxième compteur préalablement et automatiquement remis à zéro

i) la fermeture d'un circuit de mesure de la force antagoniste exercée par le piston sur l'organe pousse-piston

et en ce que ladite phase de tarage se prolonge par une phase d'injection asservie de bolus jusqu'à la vidange complète de la seringue, qui fait correspondre la position de l'organe-piston avec le zéro du premier compteur, ladite phase comprenant :

j) l'initiation de l'horloge chargée de délivrer les impulsions de démarrage du moteur, au terme d'un temps égal à la période de ladite horloge,

k) l'arrêt de la rotation du moteur avec freinage par court-circuit de l'induit lors de la coïncidence du compte du deuxième compteur avec la valeur stockée dans sa mémoire lors de l'activation de la bascule bistable.

Les caractéristiques ou avantages de l'invention apparaitront au cours de la description ci-après qui concerne des modes de réalisation préférentiels, donnés à titre non limitatif; cette description est faite en références aux dessins annexés sur lesquels:

- la figure 1 est une vue shématique des différents éléments constituants le dispositif.
- la figure 2 est une vue schématique de la face dite "face arrière".
- la figure 3 est une vue shématique de la production et du recueil des signaux élèctriques intégrés par le programmateur.
- la figure 4 représente le mode de tarage du dispositif lorsque la seringue est vide.
- la figure 5 représente le mode de détermination de la position de l'organe pousse-piston lorsque la seringue en place contient la dose à injecter.
- la figure 6 représente le diagramme de fonctionnement du dispositif à l'initiation et au cours de l'injection.
- la figure 7 représente le diagramme de fonctionnement du dispositif en fin de course.

Pour réaliser ce type de dispositif pousse-seringue, on peut le construire autour d'un ensemble électro-mécanique(1,2,3,4) coaxial contenu dans une enveloppe fermée (5).

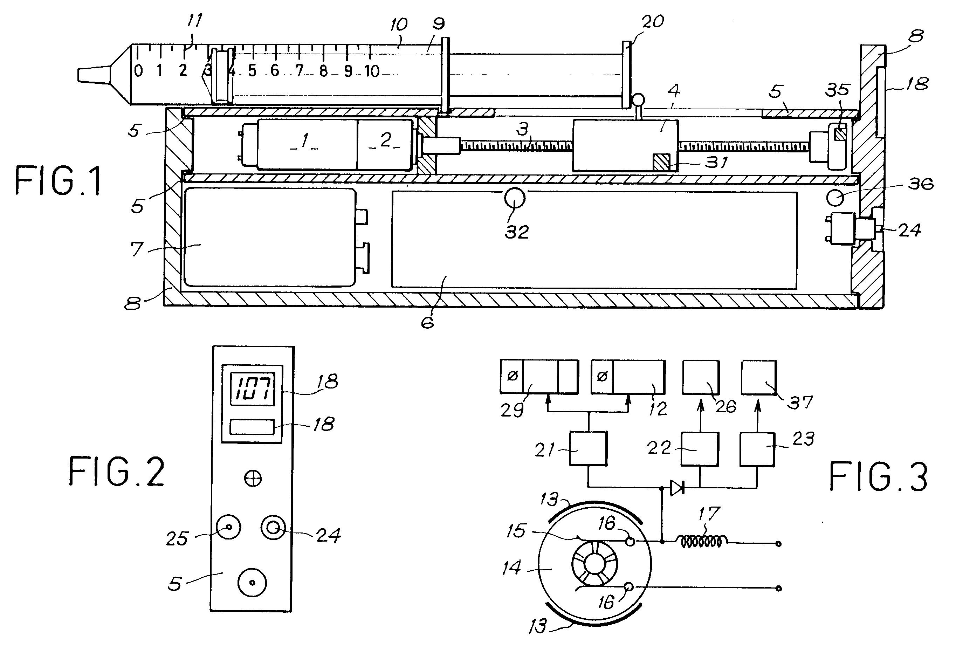

Ledit ensemble électro-mécanique ( 1,2,3,4 ) et les organes électroniques de commande (6) ainsi que la batterie d'alimentation (7) peuvent être avantageusement placés dans un boîtier (8) dont les expansions adéquatement formées maintiennent le corps de la seringue (9) par pincement comme dans le brevet WO-A-87/07843 (Figure 1)

Dans la présente invention sont décris les moyens nécessaires pour obtenir que l'angle de rotation de la vis sans fin (3) correspondant à la délivrance d'un bolus soit proportionnel au volume choisi pour une durée déterminée de traitement et contenu dans une seringue (9) de capacité nominale suffisante pour l'autonomie temporelle désirée.

Pour obtenir ce résultat, nous exposerons les données initiales suivantes :
Le volume D de solution contenu dans une seringue (9) donnée à la forme d'un cylindre de hauteur H variable et de surface de base S constante.

Or les seringues portent en général sur le cylindre (10) même des graduations (11) mesurant la hauteur H mais directement exprimée en millilitres. Le paramètre S est donc éliminé et le contrôle du débit moyen Q est réduit au contrôle de la vitesse moyenne V de la translation la hauteur H définie pour le volume D, pendant la durée totale t de vidange de la seringue (9). On peut écrire :

$$V = H/t$$

Nous avons choisi de laisser t constant quel que soit le volume D contenu dans la seringue (9). Avantageusement t est choisi égal à 24 heures ou à un multiple ou sous-multiple simple de cette durée; ainsi la fin de perfusion est prévisible et peut aisement coïncider avec les rythmes du patient et du personnel soignant.

Il est ainsi possible de faire varier D en fonction du titre de la solution en produit actif, du poids du patient ou de sa surface corporelle, de façon immédiate et pratiquement sans risque' d'erreur puisque D est affichée sur l'échelle de graduations (11) portée sur le cylindre (10) de la seringue (9). Si la précision des graduations (11) de la seringue (9) paraissait insuffisante, on pourrait par exemple peser la seringue (9) et en déduire la valeur adéquate de H correspondant au volume D choisi.

Ceci étant, et comme mentionné dans l'état de l'art, la demi-vie du produit injecté ainsi que le maintien de la perméabilité du cathéter ne permettent pas de trop longues durées de relaxation entre deux bolus consécutifs. Or, en général, ladite période de relaxation est très peu différente de t/n où n est le nombre de bolus injectés pendant le temps t. L'expérience a montré que les exigences précitées ne permettent pas d'utiliser avantageusement moins de cent bolus par 24 heures.

Le contrôle du débit moyen Q s'obtenant en faisant varier le volume du bolus, il suffira de faire adéquatement varier la translation élémentaire d correspondant à la délivrance dudit bolus de telle manière que d = H/n.

Pour rendre automatique la détermination de la translation élémentaire d = H/n, l'ensemble motoréducteur (1-2) , vis-sansfin (3) coaxiale et organe pousse-piston (4) est utilisé comme moyen intrinsèque de mesure de la hauteur H du cylindre sous la forme duquel la dose D à injecter est contenue dans la seringue (9) placée dans le porte seringue du dispositif.

Pour que le dispositif soit informé de la position instantanée de l'organe pousse-piston (4), on peut utiliser un potentiomètre dont le curseur est solidaire de l'organe poussepiston (4) ou tout autre système analogue.

Dans l'exemple décrit ci-dessous, il a été choisi préférentiellement d'utiliser un compteur décompteur réversible (12) dont la capacité correspond à toute la course mécanique possible de l'organe pousse-piston (4) et qui intègre les tours ou fractions de tours du moteur (1).

Avantageusement, les tours ou fractions de tour positifs et négatifs de la vis-sans-fin (3) sont déduits des tours ou fractions de tour du moteur (1) qui l'entraîne via un réducteur (2) de rapport connu. Les tours sont dits négatifs dans le sens de vidange de la seringue (9) et positifs dans le sens inverse.

Les signaux comptés dans le compteur décompteur reversible (12) correspondent à des tours ou fractions de tour du moteur (1).

Ils peuvent être obtenus à partir d'un système came-contacteur (38) ou d'un système optique (38) ou encore au moyen d'un aimant tournant (38) agissant à distance sur un élément sensible au magnétisme.

Préférentiellement, avec un servo-moteur (1) à faible inertie sans fer tournant à excitation par aimant permanent (13), on peut recueillir les variations de l'intensité traversant l'induit (14) engendrées par la mise en court circuit de deux lames consécutives du collecteur (15) par les balais (16). Les variations d'intensité seront recueillies aux bornes d'une résistance (17) mise en série avec l'induit (14).

Avantageusement on sépare la composante alternative, par exemple avec un condensateur. Le nombre de signaux émis par tour du moteur est égal au produit du nombre impair de lames (15) du collecteur par le nombre pair de balais (16). Les signaux (38) sont ensuite mis en forme par exemple avec un trigger de Schmidt. La polarité des signaux donne aussi le sens de rotation du moteur (Figure 3).

Avantageusement et non limitativement, la situation numérique de l'organe pousse-piston (4) contrôlée par le compteur décompteur réversible (12) est visualisée par un affichage (18) à faible consommation, par exemple à cristaux liquides, affichage qui aura en outre l'avantage de signaler par son extinction la coupure générale de l'alimentation, par exemple lors de l'activation d'un circuit de sécurité (Figure 2). Ce même affichage peut avantageusement indiquer sur commande l'état de charge-décharge de la batterie d'alimentation par visualisation de l'intégration de l'intensité reçue ou fournie.

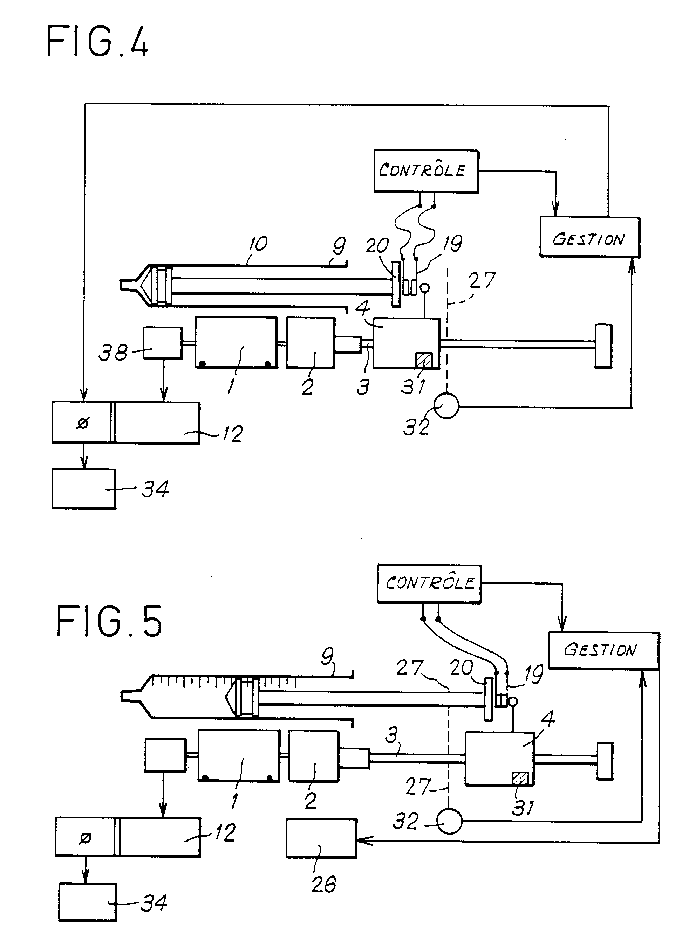

La position de l'organe pousse-piston (4) est enregistrée consécutivement lorsque la seringue (9) est totalement vide, puis lorsque la seringue (9) est remplie à la dose volumétrique choisie. Dans les deux cas, l'enregistrement de la position instantanée de l'organe pousse-piston (4) est commandé par des moyens de détection (19) du contact physique de l'organe poussepiston (4) et du piston (20) de la seringue (9) (Figure 5).

Pour déterminer le contact physique de l'organe pousse-piston (4), et du piston (20), on peut utiliser des palpeurs de modèle connu. Il est préférable d'exploiter la variation de l'intensité du courant traversant l'induit (14) du moteur (1).

Une tension représentative de ladite intensité peut avantageusement être recueillie aux bornes de la résistance (17) ou inductance en série avec l'induit (14) et préalablement décrite.

Les signaux provenant des bornes de ladite résistance (17) ou inductance sont discriminés en amplitude et en fréquence. Ainsi peuvent être distinguées des variations de premier ordre (21) ou ondes rapides fonction de la vitesse et du sens de rotation du moteur déjà mentionnées plus haut et des variations de deuxième ordre (22) ou ondes lentes correspondant à la variation de la valeur des forces de frottement (Figure 3).

En effet, l'intensité traversant l'induit est proportionnelle aux forces de frottement à vide d'une part, auxquelles s'ajoutent, lors du contact physique de l'organe pousse-piston (4) celles du piston (20) sur le cylindre (10) de la seringue (9) ainsi qu'aux frottements de la solution dans les tubulures d'écoulement.

Un troisième ordre (23) maximal de variations de ladite intensité apparaît lorsque la pression du fluide contenu dans la seringue (9) devient exessive par suite d'une entrave à l'écoulement dudit fluide, par exemple par obturation ou plicature des tubulures d'injection et/ou obturation thrombotique du cathéter, ou encore par entrave mécanique à l'avance du piston (20) notamment lorsqu'il vient en butée sur le fond du cylindre (10), ou enfin par l'apparition d'une contre-pression intravasculaire.

A chaque lancement du moteur (1) en marche avant, la pointe d'intensité correspondant à l'énergie cinétique de lancement est éliminée à l'aide d'un système de temporisation agissant sur les ondes de deuxièmes (22) et troisième ordre (23).

L'opérateur dispose d'une commande manuelle fugace pour la mise en marche continue et rapide du moteur (1) : avant (24), correspondant au sens de vidange de la seringue (9) et arrière (25). La marche arrière est toujours activable, même lorsque l'alimentation générale est interrompue. La marche avant permet d'obtenir le contact physique de l'organe pousse-piston (4) et du piston (20) dans toutes les positions possibles dudit piston (20) à l'intérieur de la course maximale. Ladite longueur de course maximale est divisée en deux segments. L'un, à l'extrémité avant de la course mécanique, est choisi de telle manière que l'organe pousse-piston (4) en contact physique avec le piston (20) d'une seringue (9) vide placée sur le dispositif soit toujours situé dans ledit segment avant, quel que soit le modèle de seringue (9) choisi. En général ledit segment avant correspond au dixième de la course maximale, ce qui limite avantageusement la dose minimale volumétrique injectable au dixième environ de la capacité nominale de la seringue (9) (Figure 4).

La détection du franchissement de la frontière (27) entre le segment court avant et le segment long arrière peut par exemple être obtenue au moyen d'un aimant permanent (31) solidaire de l'organe pousse-piston (4) agissant à distance sur un organe sensible au magnétisme (32).

Il va maintenant être décrit les opérations de mesure de la hauteur H de la dose D contenue dans la seringue (9), lesdites opérations étant considérées comme un tarage du dispositif préalable à l'injection.

L'opérateur place la seringue (9) totalement vide, piston (20) en butée sur le fond du cylindre, (10) dans l'organe de contention du dispositif, après avoir reculé si nécessaire l'organe pousse-piston (4) au moyen de la commande manuelle arrière (25).

Il déplace ensuite l'organe pousse-piston au moyen de la commande manuelle avant (24) jusqu'à l'obtention du contact de l'organe pousse-piston (4) et du piston (20) de la seringue (9). Ledit contact physique, qui survient toujours dans le segment court avant, commande automatiquement la mise au zéro du compteur décompteur réversible (12), laquelle mise à zéro provoque la coupure générale de l'alimentation, à l'exception de la marche arrière rapide à commande manuelle (Figure 4).

L'opérateur renvoit immédiatement l'organe pousse-piston (4) dans le segment long arrière à une distance suffisante pour permettre l'introduction de la seringue (9).

La seringue (9) est alors remplie à la dose choisie puis replacée sur l'organe de contention du dispositif.

L'opérateur procède alors à la mise en marche avant rapide manuelle du moteur (1). Lorsqu'apparait le contact physique de l'organe pousse-piston (4) et du piston (20), obligatoirement dans le segment long arrière, une bascule bistable (26) passe de la position repos à la position travail (Figure 5). Ledit passage déclenche immédiatement et séquentiellement les opérations suivantes :

1 - arrêt de l'alimentation du moteur (1) via l'avance rapide continue.

2 - transfert de la lecture du compteur-décompteur réversible (12) préalablement divisée par le produit du nombre total de bolus n par un facteur P fonction du rapport de réduction du réducteur (2) et du nombre de signaux (38) émis par tour ou fractions de tour du moteur (1), dans la mémoire d'un compteur préconfigurable (29).

3 - Fermeture d'un circuit tel que les signaux à provenir de la rotation du moteur (1) seront ultérieurement dirigés, parallèlement au compteur décompteur réversible (12) sur le compteur préconfigurable (29) préalablement et automatiquement remis à zéro.

4 - Fermeture d'un circuit de mesure de la force antagoniste exercée par le piston (20) sur l'organe pousse-piston (4).

5 - Initiation de l'horloge (28) consécutif au basculement du bistable (26) va initier la rotation du moteur (1) au terme d'un temps égal à la période de ladite horloge (28).

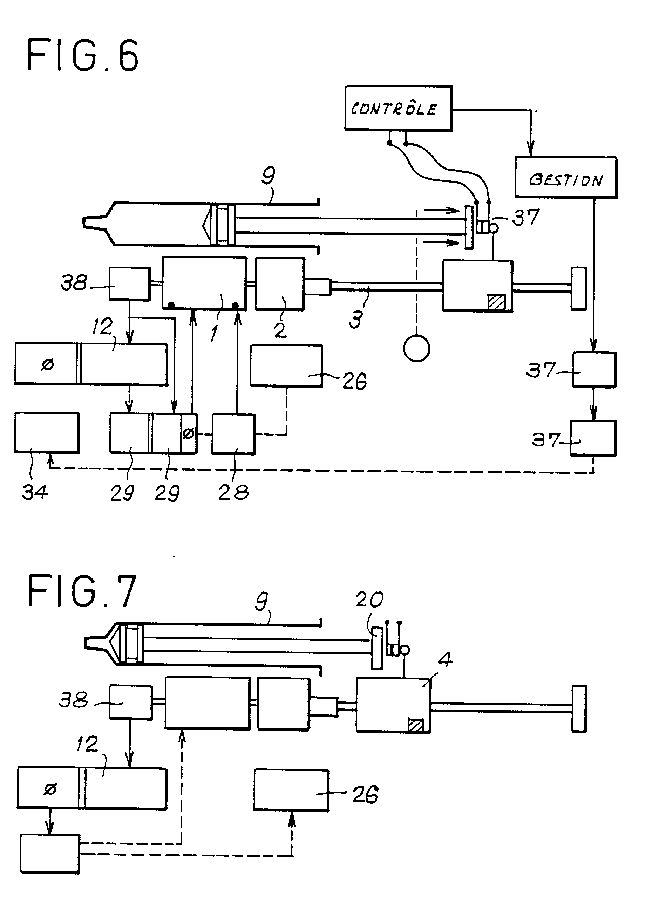

L'arrêt de ladite rotation du moteur (1) avec freinage par mise en court circuit de l'induit (14) est obtenu lors de la coïncidence du compte du compteur préconfigurable (29) avec le compte transféré dans sa mémoire lors du basculement du bistable (26) selon les modalités précitées (Figure 6). La délivrance des bolus se poursuit ensuite jusqu'à vidange totale de la seringue (9) (Figure 7). La position de l'organe poussepiston (4) correspond alors au zéro du compteur décompteur réversible (12), ce qui provoque la coupure générale de l'alimentation (34), exceptée la marche arrière rapide manuelle. Cette position est conservée ainsi indéfiniment en mémoire et permettra de supprimer ultérieurement la partie du tarage correspondant à la seringue vide tant que le même type de seringue sera utilisé.

Deux circuits de sécurité avec avertisseur sonore sont prévus.

Le premier (37) signale l'apparition d'une pression excessive et/ou l'arrêt de la translation du piston (20) provoque une augmentation brutale de l'intensité traversant l'induit (14) c'est-à-dire une variation de troisième ordre (23) comme décrit précédement. Avec la bascule (26) en position travail correspondant au cours de l'injection normale, ladite variation de troisième ordre (23) déclenche un avertisseur sonore. Si l'opérateur ne procède pas à la levée de l'obstacle, une temporisation coupe l'alimentation générale après un délai de l'ordre de cinq minutes, ce qui a pour effet de mettre le compteur décompteur réversible (12) à zéro. Une coupure générale de l'alimentation alors que la seringue (9) n'est pas totalement vide témoigne à postériori de l'incident et la position du piston (20) de son heure.

Le deuxième circuit de sécurité protège contre la distribution immédiatement consécutive de deux bolus de volume maximum sans période de relaxation intermédiaire.

A cet effet, la rotation de la vis-sans-fin (3) est directement intégrée dans un compteur spécial de telle manière que l'angle de rotation correspondant à la délivrance desdits deux bolus provoque immédiatement l'arrêt du moteur (1) par coupure générale et totale de l'alimentation et avantageusement l'excitation d'un affichage visuel dit de danger de manière à interdire la remise en route de l'appareil.

Avec un choix judicieux du pas de la vis-sans-fin, le bolus de volume maximal peut être délivré avec moins d'un tour de la vis-sans-fin (3). Le compteur distinct signalera en conséquence tout dépassement d'un angle de rotation égal à deux tours à chaque initiation de marche de l'horloge (28) (Figure 1).

Sinon, ledit compteur retournera à zéro. Avantageusement et non limitativement, la détection de la rotation de la vis-sans-fin peut être obtenue par aimant (35) fixé sur celle-ci et agissant à distance sur élément sensible au magnétisme (36), comme dans le brevet WO-A-87/07843.

## Revendications

1. Dispositif pousse-seringue pour traitement ambulatoire au moyen d'une seringue (9) indifférenciée munie d'un piston (20), délivrant une pluralité de volumes élémentaires ou bolus séparés par des périodes de relaxation, comprenant :

- un boîtier (8) dans lequel est située une enveloppe-guide fermée (5) contenant un ensemble électromécanique' composé d'un moteur (1) à faible inertie sans fer tournant, à excitation par aimant permanent,
- un réducteur (2),
- une vis-sans-fin (3) coaxiale sur laquelle se déplace un chariot porteur d'un organe pousse-piston (4),
- une source d'énergie électrique (7), et,
- des circuits électroniques de contrôle avec au moins un compteur,
caractérisé en ce qu'il comprend en outre :
- un premier compteur-décompteur réversible (12) qui intègre les tours positifs et négatifs du moteur,
- un second compteur préconfigurable (29),
- un moyen qui divise la longueur totale de la course en un segment avant et un segment arrière,

- un moyen de détection du contact entre l'organe pousse-piston (4) et le piston (20),
- un circuit capable de mettre à zéro le premier compteur-décompteur (12) lors de la détection d'un contact entre l'organe pousse-piston et le piston,
- un circuit capable de transmettre, lors de la détection d'un contact entre l'organe pousse-piston (4) et le piston (20) dans le segment arrière, la lecture du compteur-décompteur réversible (12) au compteur préconfigurable (29) après division préalable par le produit nP du nombre total de bolus n et d'un facteur P fonction à la fois du rapport de réduction du réducteur (2) et du nombre de signaux (38) émis par tour de moteur (1),
- un circuit qui permet de diriger les signaux provenant de la rotation du moteur sur les deux compteurs (12,29) à la fois,
- une horloge qui permet d'initialiser la rotation du moteur au terme d'un temps égal à la période de cette horloge,
- un circuit qui permet d'arrêter la rotation du moteur lorsque la valeur comptée par le premier compteur (12) et la valeur stockée dans la mémoire du compteur (29) coïncident.

2. Dispositif selon la revendication 1, caractérisé en ce qu'il est muni de moyens d'enregistrement de la position instanta'née de l'organe pousse-piston (4) lorsque la seringue (9) est totalement vide, puis lorsque la seringue est chargée du volume choisi, de telle sorte que la distance entre ces positions de l'organe pousse-piston soit connue lors d'une opération de tarage préalable.

3. Dispositif selon l'une quelconque des revendications précédentes, caractérisé en ce qu'il comprend un moyen qui intègre les tours ou fractions de tour du moteur en recueillant les signaux engendrés par la mise en court-circuit de deux lames consécutives du collecteur (15) au passage sous un des balais.

4. Dispositif selon la revendication 3, caractérisé en ce qu'il comprend un moyen qui permet de recueillir les signaux provenant de la mise en court-circuit des lames du collecteur (15) et de les discriminer en amplitude et fréquence en au moins trois ordres de réponse (21,22,23) aux variations de l'intensité traversant l'induit (14).

5. Dispositif selon la revendication 4, caractérisé en ce qu'une résistance (17) mise en série avec l'induit (14) permet de recueillir et discriminer les variations de l'intensité le traversant.

6. Dispositif selon la revendication 5, caractérisé en ce qu'il comprend des moyens de recueillir des signaux d'au moins trois ordres (21, 22, 23) aux bornes de la résistance (17) mise en série avec l'induit (14).

7. Dispositif selon l'une quelconque des revendications précédentes, caractérisé en ce que le premier compteur-décompteur (12) intègre les signaux de premier ordre (21) des deux polarités pendant toute la durée des opérations de tarage et d'injection.

8. Dispositif selon l'une quelconque des revendications précédentes, caractérisé en ce que le premier compteur-décompteur (12) déclenche l'initiation de l'injection lorsqu'il reçoit un signal de deuxième ordre (22) alors que le piston (20) est situé dans la portion longue arrière de sa course.

9. Dispositif selon l'une quelconque des revendications précédentes, caractérisé en ce qu'un intégrateur des tours ou fractions de tour de la vis-sans-fin (3) et mécaniquement solidaire de celle-ci compte le nombre de bolus délivrés à la suite de chaque initiation en provenance de l'horloge (28).

10. Dispositif selon la revendication 9, caractérisé en ce que l'intégrateur des tours ou fractions de tour de la vis-sans-fin (3) est formé par un aimant permanent (35) tournant et fixé mécaniquement en bout de vis-sans-fin et agissant à distance sur un élément sensible au magnétisme (36).

11. Dispositif selon la revendication 10, caractérisé en ce que l'intégrateur des tours de la vis-sans-fin (3) est relié à un moyen (37) qui comprend un compteur et arrête irrévocablement le moteur et excite un signal permanent de mise hors service lorsque deux bolus consécutifs sont délivrés par la même initiation de la rotation du moteur (1).

12. Procédé de contrôle du débit moyen assuré par un dispositif pousse-seringue motorisé selon l'une des revendications 1 à 11, caractérisé en ce qu'il comprend une phase de tarage préalable telle que :
   a) la seringue (9) est placée vide dans le dispositif

b) l'organe pousse-seringue (4) est déplacé vers l'avant jusqu'à l'obtention du contact avec le piston (20)

c) le premier compteur (12) est remis à zéro lorsque ledit contact survient dans la portion courte avant de la course mécanique de l'organe pousse-piston (4), provoquant la coupure générale de l'alimentation du dispositif, à l'exception de la marche arrière rapide à commande manuelle

d) l'organe pousse-piston (4) est renvoyé dans le segment long arrière pour permettre l'introduction de la seringue (9) contenant la dose D de fluide à injecter

e) l'organe pousse-piston (4) est renvoyé en marche avant et entre en contact avec le piston de la seringue dans la portion longue arrière du dispositif de façon à activer une bascule bistable (26) qui provoque :

f) l'arrêt de l'alimentation du moteur (1) via l'avance rapide

g) le transfert de la lecture du premier compteur (12), préalablement divisée par nP correspondant au produit du nombre total de bolus n par un facteur P fonction du rapport de réduction du réducteur (2) et du nombre de signaux (38) émis par tour ou fraction de tour du moteur (1), dans la mémoire d'un deuxième compteur, préconfigurable (29)

h) la fermeture d'un circuit tel que les signaux à provenir de la rotation du moteur (1) seront ultérieurement dirigés, parallèlement au premier compteur (12) vers le deuxième compteur (29) préalablement et automatiquement remis à zéro

i) la fermeture d'un circuit de mesure de la force antagoniste exercée par le piston (20) sur l'organe pousse-piston (4)

et en ce que ladite phase de tarage se prolonge par une phase d'injection asservie de bolus jusqu'à la vidange complète de la seringue, qui fait correspondre la position de l'organe-piston (4) avec le zéro du premier compteur (12), ladite phase comprenant :

j) l'initiation de l'horloge (28) chargée de délivrer les impulsions de démarrage du moteur (1), au terme d'un temps égal à la période de ladite horloge,

k) l'arrêt de la rotation du moteur avec freinage par court-circuit de l'induit lors de la coïncidence du compte du deuxième compteur (29) avec la valeur stockée dans sa mémoire lors de l'activation de la bascule bistable (26).

**Claims**

1. Syringe-pusher device for ambulatory treatment by means of an indifferentiated syringe (9) provided with a piston (20), delivering a plurality of elementary volumes or boluses separated by periods of relaxation, comprising:
   - a casing (8) in which is located a closed guide envelope (5) containing an electromechanical assembly composed of a non-free-running, low-inertia motor (1) with energization by permanent magnet,
   - a reduction gear (2),
   - a coaxial endless screw (3) on which moves a carriage bearing a piston-pusher member (4),
   - a source of electrical energy (7), and
   - electronic control circuits with at least one counter,
   characterized in that it further comprises:
   - a first reversible counter (12) which integrates the positive and negative revolutions of the motor,
   - a second pre-configurable counter (29),
   - a means which divides the total length of the stroke into a front segment and a rear segment,
   - a means for detecting the contact between the piston-pusher member (4) and the piston (20),
   - a circuit capable of placing at zero the first reversible counter (12) upon detection of a contact between the piston-pusher member and the piston,
   - a circuit capable of transmitting, upon detection of a contact between the piston-pusher member (4) and the piston (20) in the rear segment, the reading of the reversible counter (12) to the pre-configurable counter (29) after prior division by the product nP of the total number of boluses n and a factor P function both of the ratio of reduction of the reduction gear (2) and of the number of signals (38) emitted per revolution of motor (1),
   - a circuit which makes it possible to direct the signals coming from the rotation of the motor on the two counters (12, 29) at the same time,
   - a clock which makes it possible to initialize rotation of the motor after a time equal to the period of this clock,
   - a circuit which makes it possible to stop rotation of the motor when the value counted by the first counter (12) and the value stored in the memory of counter (29) coincide.

2. Device according to Claim 1, characterized in that it is provided with means for recording the instantaneous position of the piston-pusher member (4) when the syringe (9) is totally empty, then when the syringe is loaded with the chosen volume, so that the distance between these positions of the piston-pusher member is known during a prior calibration operation.

3. Device according to either one of the preceding Claims, characterized in that it comprises a means which integrates the revolutions or fractions of revolution of the motor by collecting the signals generated by short circuiting two consecutive blades of the collector (15) on passage beneath one of the brushes.

4. Device according to Claim 3, characterized in that it comprises a means which makes it possible to collect the signals coming from short-circuiting the blades of the collector (15) and to discriminate them in amplitude and frequency in at least three orders of response (21, 22, 23) to the variations of the intensity traversing the armature (14).

5. Device according to Claim 4, characterized in that a resistor (17) placed in series with the armature (14) makes it possible to collect and discriminate the variations of the intensity traversing it.

6. Device according to Claim 5, characterized in that it comprises means for collecting the signals of at least three orders (21, 22, 23) at the terminals of the resistor (17) placed in series with the armature (14).

7. Device according to any one of the preceding Claims, characterized in that the first reversible counter (12) integrates the signals of first order (21) of the two polarities during the whole duration of the operations of calibration and of injection.

8. Device according to any one of the preceding Claims, characterized in that the first reversible counter (12) triggers off initiation of the injection when it receives a signal of second order (22) whilst the piston (20) is located in the rear long portion of its stroke.

9. Device according to any one of the preceding Claims, characterized in that an integrator of the revolutions or fractions of revolution of the endless screw (3) and mechanically fast therewith, counts the number of boluses delivered further to each initiation coming from the clock (28).

10. Device according to Claim 9, characterized in that the integrator of the revolutions or fractions of revolution of the endless screw (3) is formed by a rotating permanent magnet (35) mechanically fixed at the end of the endless screw and acting at a distance on an element (36) sensitive to magnetism.

11. Device according to Claim 10, characterized in that the integrator of the revolutions of the endless screw (3) is connected to a means (37) which comprises a counter and irrevocably stops the motor and energizes a permanent signal for putting out of action when two consecutive boluses are delivered by the same initiation of the rotation of the motor (1).

12. Process for controlling the mean flow ensured by a motorized syringe-pusher device according to one of Claims 1 to 11, characterized in that it comprises a prior calibration phase such that:

a) the syringe (9) is placed empty in the device,

b) the syringe-pusher member (4) is displaced forwardly until contact with piston (20) is obtained,

c) the first counter (12) is returned to zero when said contact occurs in the front short portion of the mechanical stroke of the piston-pusher member (4), provoking general cut-off of the supply of the device, except for manually controlled rapid reverse motion,

d) the piston-pusher member (4) is returned in the rear long segment to allow introduction of the syringe (9) containing the dose D of fluid to be injected,

e) the piston-pusher member (4) is returned into forward motion and comes into contact with the piston of the syringe in the rear long portion of the device so as to activate a bistable trigger circuit (26) which provokes:

f) stop of the supply of the motor (1) via the rapid advance,

g) transfer of the reading of the first counter (12), previously divided by nP corresponding to the product of the total number of boluses n by a factor p function of the ratio of reduction of the reduction gear (2) and the number of signals (38) emitted by each or fraction of revolution of the motor (1), in the memory of a second pre-configurable counter (29),

h) closure of a circuit such that the signal to come from the rotation of the motor (1) will subsequently be directed, parallel to the first counter (12) towards the second counter (29) previously and automatically returned to zero,

i) closure of a circuit for measuring the antagonistic force exerted by the piston (20) on the piston-pusher member (4),

and in that said phase of calibration is extended by a servo-controlled phase of injection of boluses up to complete emptying of the syringe, which causes the position of the piston member (4) to correspond to the zero of the first counter (12), said phase comprising:

j) initiation of the clock (28) charged with delivering the pulses for starting up the motor (1), after a time equal to the period of said clock,

k) stop of the rotation of the motor with braking by short circuit of the armature upon coincidence of the count of the second counter (29) with the value stored in its memory upon activation of the bistable trigger circuit (26).

**Patentansprüche**

1. Spritzenschubvorrichtung zur ambulanten Behandlung mittels einer mit einem Kolben (20) versehenen beliebigen Spritze (9), die eine Mehrzahl von Elementarmengen oder Boli, unterbrochen durch Entspannungsperioden, abgibt, umfassend:
    - ein Gehäuse (8) in dem sich eine geschlossene Führungshülse (5) befindet, die eine elektromechanische Einheit enthält, welche durch einen Motor (1) mit geringer Trägheit ohne Eisenkern, mit Erregung durch einen Permanentmagneten, gebildet ist,
    - eine Reduktionseinrichtung (2),
    - eine koaxiale Schraubenspindel (3), auf der ein Tragwagen für ein Kolbenschuborgan (4) verschiebbar ist,
    - eine elektrische Energiequelle (7), und
    - elektronische Steuerkreise mit mindestens einem Zähler,
        dadurch gekennzeichnet, daß sie außerdem umfaßt:
    - einen ersten, umschaltbaren Vorwärts-Rückwärts-Zähler (12), der die positiven und negativen Umdrehungen des Motors integriert,
    - einen zweiten, vorkonfigurierbaren Zähler (29),

    - ein Mittel, das die Gesamtlänge des Hubs in einen vorderen Abschnitt und einen hinteren Abschnitt unterteilt,
    - ein Mittel zum Detektieren des Kontakts zwischen dem Kolbenschuborgan (4) und dem Kolben (20),
    - eine Schaltung, die den ersten Vorwärts-Rückwärts-Zähler (12) bei der Detektion eines Kontaktes zwischen dem Kolbenschuborgan und dem Kolben auf Null stellen kann,
    - eine Schaltung, die bei der Detektion eines Kontaktes zwischen dem Kolbenschuborgan (4) und dem Kolben (20) im hinteren Abschnitt die Ablesung des umschaltbaren Vorwärts-Rückwärts-Zählers (12) an den vorkonfigurierbaren Zähler (29) nach vorheriger Division durch das Produkt nP aus der Gesamtzahl der Boli n und einem Faktor P als Funktion gleichzeitig des Reduktionsverhältnisses der Reduktionseinrichtung (2) und der Anzahl an pro Umdrehung des Motors (1) gesendeten Signalen (38) übermitteln kann,
    - eine Schaltung, die die Lenkung der von der Drehung des Motors ausgehenden Signale an beide Zähler (12,29) auf einmal gestattet,
    - einen Zeitgeber, der die Initialisierung der Drehung des Motors zum Term einer Zeit gleich der Taktdauer dieses Zeitgebers gestattet,
    - eine Schaltung, die den Stopp der Drehung des Motors gestattet, wenn der vom ersten Zähler (12) gezählte Wert und der im Speicher des Zählers (29) gespeicherte Wert übereinstimmen.

2. Vorrichtung nach Anspruch 1, dadurch gekennzeichnet, daß sie mit Mitteln zum Aufzeichnen der momentanen Position des Kolbenschuborgans (4), wenn die Spritze (9) vollkommen leer ist, und dann, wenn die Spritze mit der gewählten Menge gefüllt ist, versehen ist, so daß der Abstand zwischen diesen Positionen des Kolbenschuborgans bei einer Vortarierung bekannt ist.

3. Vorrichtung nach einem der vorhergehenden Ansprüche, dadurch gekennzeichnet, daß sie ein Mittel umfaßt, das die Umdrehungen oder Umdrehungsanteile des Motors unter Aufnahme der Signale integriert, die durch die Kurzschlußschaltung zweier aufeinanderfolgender Lamellen des Kollektors (15) heim Durch:ritt unter einer der Bürsten erzeugt werden.

**4.** Vorrichtung nach Anspruch 3, dadurch gekennzeichnet, daß sie ein Mittel umfaßt, das die Aufnahme der von der Kurzschlußschaltung der Lamellen des Kollektors (15) ausgehenden Signale und die Trennung derselben in Amplitude und Frequenz in mindestens drei Ordnungen (21, 22, 23) der Reaktion auf die Veränderungen der durch den Anker (14) strömenden Energiefluenz gestattet.

**5.** Vorrichtung nach Anspruch 4, dadurch gekennzeichnet, daß ein mit dem Anker (14) in Serie geschalteter Widerstand (17) die Aufnahme und Trennung der Veränderungen der durch ihn strömenden Energiefluenz gestattet.

**6.** Vorrichtung nach Anspruch 5, dadurch gekennzeichnet, daß sie Mittel zur Aufnahme von Signalen von mindestens drei Ordnungen (21,22,23) an den Anschlußklemmen des mit dem Anker (14) in Serie geschalteten Widerstandes (17) umfaßt.

**7.** Vorrichtung nach einem der vorhergehenden Ansprüche, dadurch gekennzeichnet, daß der erste Vorwärts-Rückwärts-Zähler (12) die Signale erster Ordnung (21) der beiden Polaritäten während der gesamten Dauer der Tarier- und Injektionsvorgänge integriert.

**8.** Vorrichtung nach einem der vorhergehenden Ansprüche, dadurch gekennzeichnet, daß der erste Vorwärts-Rückwärts-Zähler (12) den Start der Injektion auslöst, wenn er ein Signal zweiter Ordnung (22) empfängt, während sich der Kolben (20) im hinteren, langen Abschnitt seines Hubs befindet.

**9.** Vorrichtung nach einem der vorhergehenden Ansprüche, dadurch gekennzeichnet, daß ein Integrator für die Umdrehungen oder Umdrehungsanteile der Schraubenspindel (3), der mechanisch fest mit dieser verbunden ist, die Anzahl an Boli zählt, die infolge jedes vom Zeitgeber (28) ausgehenden Starts abgegeben werden.

**10.** Vorrichtung nach Anspruch 9, dadurch gekennzeichnet, daß der Integrator für die Umdrehungen oder Umdrehungsanteile der Schraubenspindel (3) durch einen Permanentmagneten (35) gebildet ist, der sich dreht und mechanisch am Ende der Schraubenspindel fixiert ist und aus einer Entfernung auf ein für Magnetismus empfindliches Element (36) wirkt.

**11.** Vorrichtung nach Anspruch 10, dadurch gekennzeichnet, daß der Integrator für die Umdrehungen oder Umdrehungsanteile der Schraubenspindel (3) an ein Mittel (37) angeschlossen ist, welches einen Zähler umfaßt und den Motor unwiderruflich stoppt und ein permanentes Signal zur Außerbetriebnahme erregt, wenn zwei aufeinanderfolgende Boli durch ein- und denselben Start der Drehung des Motors (1) abgegeben werden.

**12.** Verfahren zur Steuerung der durch eine mit Motor versehene Spritzenschubvorrichtung nach einem der Ansprüche 1 bis 11 gewährleisteten mittleren Durchflußmenge, dadurch gekennzeichnet, daß es eine Phase zur Vortarierung aufweist derart, daß

a) die Spritze (9) leer in der Vorrichtung plaziert wird,

b) das Kolbenschuborgan (4) nach vorne verschoben wird, bis ein Kontakt mit dem Kolben (20) erreicht wird,

c) der erste Zähler (12) auf Null gestellt wird, wenn der Kontakt im vorderen, kurzen Abschnitt des mechanischen Hubs des Kolbenschuborgans (4) erfolgt, wodurch die Generalunterbrechung der Speisung der Vorrichtung bewirkt wird, mit Ausnahme des raschen Rücklaufs mit manueller Steuerung,

d) das Kolbenschuborgan (4) in den hinteren, langen Abschnitt zurückgebracht wild, um die Einführung der Spritze (9) zu ermöglichen, die die zu injizierende Dosis D an Fluid enthält,

e) das Kolbenschuborgan (4) wieder in den Vorlauf gebracht wird und in Kontakt mit dem Kolben der Spritze im hinteren, langen Abschnitt der Vorrichtung gelangt, um ein bistabiles Kippglied (26) zu aktivieren, welches folgendes bewirkt:

f) den Stopp der Beaufschlagung des Motors (1) über den Schnellgang

g) die Übertragung der Ablesung des ersten Zählers (12), die zuvor durch nP entsprechend dem Produkt der Gesamtzahl an Boli n mit einem Faktor P als Funktion des Reduktionsverhältnisses der Reduktionseinrichtung (2) und der Anzahl an pro Umdrehung oder Umdrehungsanteil des Motors (1) gesendeten Signale (38) dividiert wurde, in den Speicher eines zweiten, vorkonfigurierbaren Zählers (29),

h) die Sperrung einer Schaltung derart, daß die von der Drehung des Motors (1) auszugehenden Signale später, parallel zum ersten Zähler (12), zum zweiten Zähler (29) gelenkt werden, der zuvor automatisch auf Null gestellt wurde,

i) die Sperrung einer Schaltung zum Messen der Gegenkraft, die vom Kolben (20) auf das Kolbenschuborgan (4) ausgeübt wird,

und daß die Tarierphase durch eine angetriggerte Bolusinjektionsphase verlängert wird bis zur vollständigen Entleerung der Spritze, die die Position des Kolbenschuborgans (4) mit dem Nullwert des ersten Zählers (12) übereinstimmen läßt, welche Phase umfaßt:

j) den Start des Zeitgebers (28), dessen Aufgabe es ist, die Anfahrimpulse des Motors (1) zum Term einer Zeit gleich der Taktdauer des Zeitgebers abzugeben,

k) den Stopp der Drehung des Motors mit Bremsung durch Kurzschluß des Ankers bei Übereinstimmung der Zählung des zweiten Zählers (29) mit dem in seinem Speicher gespeicherten Wert bei der Aktivierung des bistabilen Kippglieds (26).

FIG.1

FIG.2

FIG.3

# FIG.4

CONTRÔLE

GESTION

10  9  20  19  27

38  4  31  32

1  2  3

∅  12

34

# FIG.5

CONTRÔLE

GESTION

9  27  20  19

3  4

27  31  32

1  2

∅  12  26

34

# FIG.6

CONTRÔLE

GESTION

9

37

38

12

ø

1    2    3

26

34    29    29    28

37

37

# FIG.7

9    20

38    4

12

ø

26